# EUROPEAN PATENT APPLICATION

(11) **EP 2 405 274 A1**
(43) Date of publication of application: **11.01.2012**
(21) Application number: 10168538.6
(22) Date of filing: 06.07.2010
(51) Int. Cl.: G01N 33/86, C12Q 1/56

(54) **Monitoring anticoagulant therapy and identifying reversible direct factor Xa inhibitors**

(71) Applicant: Good biomarker Sciences B.V., 2171 CD Sassenheim (NL)
(72) Inventor: Kluft, C., 2171 CD, Sassenheim (NL)
(74) Representative: Jorritsma, Ruurd

(57) **Abstract**

The invention pertains to the use of an RVV-V sensitive blood clotting assay for monitoring anticoagulant therapy comprising reversible direct factor Xa inhibitor(s) such as rivaroxaban, and/or pre-operative screening for such reversible direct factor Xa inhibitors. The invention also pertains to a blood clotting assay kit-of-parts, comprising (a) a first container comprising RVV-V sensitive thromboplastin reagent; and (b) a second container comprising RVV-V insensitive thromboplastin reagent(s) or RVV-V, in either case optionally together with RVV-V sensitive thromboplastin reagent(s).

## Description

### FIELD OF THE INVENTION

The present invention is in the field of monitoring anticoagulant therapy involving reversible direct factor Xa inhibitors, and pre-operative screening for and identifying of reversible direct factor Xa inhibitors. Insofar as the invention concerns anticoagulant drug therapy, it concerns therapy based on reversible direct factor Xa inhibitor(s).

### BACKGROUND OF THE INVENTION

Recently, a Factor Xa inhibitor called Rivaroxaban is approved in the EU and several other countries for venous thromboembolism prevention after hip and knee replacement surgery, and is in advanced clinical development for chronic indications.

Coagulation inhibitors directed to factor Xa are not new, since heparins have factor Xa as one of their targets. Heparins and heparinoids act by complexing with antithrombin and thus accelerating its irreversible inhibition properties. Tests for heparinoids recording the factor Xa action of heparinoids are available, and include clotting tests such as PiCT® and Heptest®. These anti-Xa clotting tests are unfortunately not available for routine laboratories yet.

Alternatively, various laboratory clotting test assays are commercially available for detecting bleeding disorders and/or monitoring irreversible anticoagulants, making use of the 'prothrombin time' (PT) or the 'activated partial thromboplastin time' (APTT) according to Quick. The times to establish blood clotting after addition of a certain thromboplastin reagent thus measured are typically converted to the PT ratio, i.e. the patient's PT value divided by the mean PT of the laboratory's normal population range. The different diagnostic tests on the market mainly differ in the prothrombin time reagent applied, Innovin (Dade Behring), Neoplastin CI Plus (Stago), Thromborel S (Dade Behring), Recombiplastin (IL) and Neoplastin CI (Stago) being most favoured in the field. However, the thromboplastins differ greatly in their sensitivity to individual coagulation factors, so that the same sample from a patient can have different prothrombin times when tested with reagents from different manufacturers. In the art ways have been accomplished to achieve standardized results of the PT, the so-called international normalized ratio (INR).

The therapeutic range of anticoagulant therapy is based on the avoidance of bleeding and thrombotic complications. When monitoring anticoagulant therapy, an elongation of prothrombin time by a factor of 2 is most desirable for long term therapy (O'Reilly, "Hemostasis and Thrombosis, Basic Principles and Clinical Practice", Coleman, eta 1., eds., J.B. Lippencott Co. Philadelphia, pp. 1367 - 1372, 1987. Accepted overall therapeutic ranges for anticoagulant therapy are between INR 2-5, corresponding to clotting times of about 12-80 seconds.

In the art it is claimed that rivaroxaban can be used at a fixed dose without the need for monitoring, due to its predictable pharmacokinetics across a wide spectrum of patients and its flat dose response. Yet a quantitative determination of rivaroxaban might be valuable in some cases. It would for instance still be required to detect rivaroxaban in patients before medical interventions to prevent creating an unnecessary and unwanted bleeding risk. However, as it appears, the existing assays used in routine laboratories struggle with detecting the new generation reversible coagulation inhibitors. The influence of rivaroxaban on PT in numerous clotting test assays is for instance addressed by Samama et al. "Effects of rivaroxaban and fondaparinux on clotting assays", Poster P173 presented at 20th Int. Congress on Thrombosis (ICT), Athens, Greece; 25 - 28 June 2008, and Samama et al. "Effects of the novel, oral, direct Factor Xa inhibitor Rivaroxaban on coagulation assays", abstract 3028 presented at the American Society of Hematology (ASH) 50th Annual Meeting and Exposition, San Francisco, CA, USA; December 6-9, 2008. Concentration-dependent PT ratios were found between 1.0 and 2.1. This is confirmed in a PT test on rivaroxaban using a large number of commercially available kits in example 1 attached.

Hence, the art is still in need of an optimum clotting assay for rivaroxaban, a screening method to be implemented in routine laboratories, ready to identify individuals at all times of day and week of having rivaroxaban on board.

### SUMMARY OF THE INVENTION

It is an object of the invention to provide reliable clotting assays which are sensitive to reversible direct factor Xa inhibitors such as Rivaroxaban and Apixaban, and which can be implemented in routine laboratories. Those PT assays exhibiting an upper limit of the PT ratio (prolonged PT time at 600 µl/ml rivaroxaban over reference PT value) of at least 3.0, more preferably at least 3.5, in particular at least 4.0 are most promising. Otherwise, coverage of the therapeutic range is considered unsatisfactory.

It is preferred that the PT reference (in absence of any reversible and irreversible blood clotting inhibitors) is less than 20 seconds, more preferably less than 18 seconds. Extended reference blood clotting times may obscure the effects of the reversible direct factor Xa inhibitors on PT.

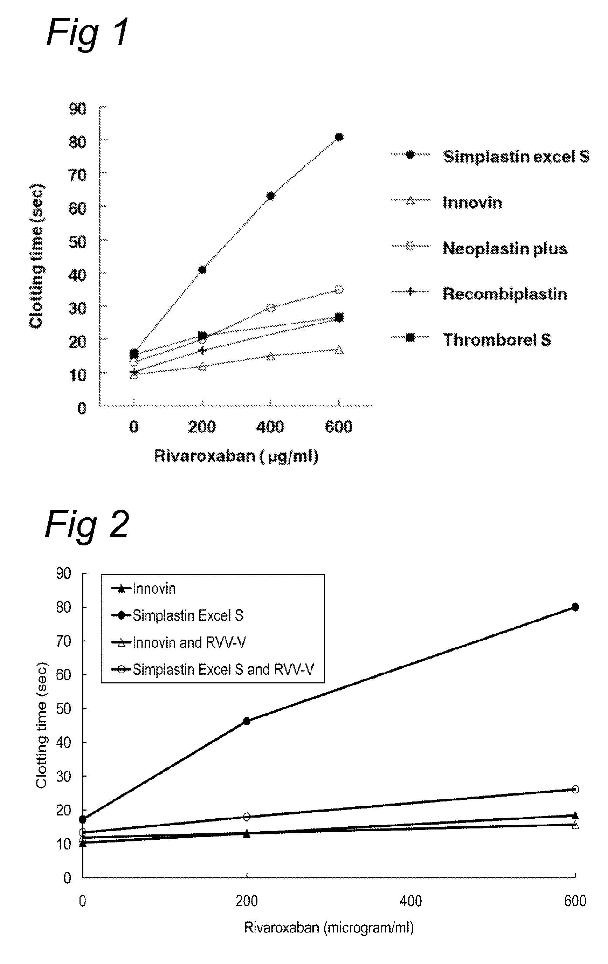

In an *in vitro* study on rivaroxaban, the inventors found among a wide range of tested PT reagents one reagent by the name of Simplastin Excel S or Triniclot Excel S that behaved different compared to others: where most existing assays failed to provide PT within therapeutically accepted ranges and thus confirmed observations in the field, the Simplastin Excel S and/or Triniclot Excel S surprisingly showed high sensitivity for rivaroxaban, within the therapeutic range (i.e. ratio window 2 - 5). The evidence is found in Figure 1 and Table 1.

Although the inventors do not wish to be tied down to any theory, the effect is believed to be linked to factor V activation being the rate-limiting step in this particular assay. Based on that finding, the inventors concluded that factor V sensitive clotting assays are particularly suited for testing for reversible factor Xa inhibitors. The effect of rivaroxaban on factor Va formation is strongly underestimated. Although thromboplastin reagents have been marketed for years and known to differ in sensitivity to individual coagulation factors , up to present the factor V activation sensitivity of thromboplastin reagents has not attracted much attention, let alone has there been made a link between the new generation reversible anticoagulants and the fact that anticoagulants become more effective when factor Va formation becomes rate limiting.

Of the at least 20 different thromboplastins commercially marketed (see e.g. examples) it is now found that the skilled person can readily select those appropriate for screening, detecting and identifying the reversible anticoagulants being sensitive to the rate of factor Va formation, i.e. those thromboplastins for which factor Va formation predominantly rules the clotting time. Thereto, the skilled person can make use of well-established properties of Factor V-activating enzyme from Russell's Viper Venom (RVV-V) applied in the field. RVV-V can speed up the rate of factor Va formation and thus renders PT assays independent of this aspect when added. Knowing that only RVV-V "sensitive" PT assays are suited for measuring the reversible anticoagulants, the skilled person simply has to determine whether or not the addition of RVV-V to a PT and a sample with reversible direct factor Xa inhibitors such as Rivaroxaban results in a detectable decrease in PT. The addition of RVV-V should remove the PT's sensitivity for Factor V activation. As an example, the effect of RVV-V on Rivaroxaban-prolonged PT using Simplastin Excel S is shown in the accompanying examples: time is reduced from 80 to 30 seconds upon addition of RVV-V. For sake of comparison, it is also illustrated that no appreciable acceleration is observed when using blood clotting assays based on Innovin (Dade Behring)..

In one aspect, the invention thus pertains to the use of RVV-V sensitive blood clotting assays for monitoring anti-coagulant therapy involving reversible direct factor Xa inhibitors.

In another aspect, the invention pertains to a blood clotting assay kit comprising a first container with RVV-V sensitive thromboplastin reagent(s); and a second container with RVV-V insensitive thromboplastin reagent(s) or RVV-V, in order to identify reversible direct factor Xa inhibitors such a s Rivaroxaban, for instance in pre-operative screening. The second container may comprise RVV-V sensitive thromboplastin reagent in addition to the RVV-V or RVV-V insensitive thromboplastin reagent(s). When planning medical interventions, it is of the essence to be able to distinguish between the different anticoagulant inhibitors, given the fact that the blood clearance for irreversible vitamin K antagonists and the new generation of reversible inhibitors takes about 3 and 1 days, respectively. Unwanted bleeding risks are thus avoided.

### LIST OF FIGURES

Figure 1 shows the effect of rivaroxaban on PT for different blood clotting assays;
Figure 2 shows the effect of RVV-V on rivaroxaban-prolonged PT in Simplastin Excel S assay;
Figure 3 shows the clotting intervals of plasma spiked with rivaroxaban as observed with an RVV-V sensitive and with an RVV-V insensitive tissue thromboplastin reagent, and the PT in case of a 1:1 mixture of RVV-V sensitive and insensitive reagents.

### DETAILED DESCRIPTION OF THE INVENTION

"Reversible direct factor Xa inhibitor" stands for those antithrombotics which act directly upon Factor Xa in the coagulation cascade, without the need for antithrombin as a mediator. These antagonists reversibly inhibit thrombin generation by both the intrinsic and the tissue factor or extrinsic pathways, and distinguish from the class of irreversible Xa inhibitors which act through the irreversible complexation with a cofactor such as antithrombin. Examples are Rivaroxaban, Apixaban, Otamixaban, YM466. Rivaroxaban is marketed as Xarelto®.

"Prothrombin time" is abbreviated as PT and refers to the time interval between the addition of a thromboplastin or prothrombin time reagent and the appearance of a clot in platelet poor, citrated plasma. In the context of the invention, the term "reagent" is short for "thromboplastin", "prothrombin time reagent" or "thromboplastin reagent". The test is sometimes also referred to as Quick's test. "Prothrombin ratio" is abbreviated as PR and refers to the prothrombin time of an individual's plasma (either normal or abnormal) divided by the prothrombin time of pool of normal individual plasmas. Here, the test referred to are in accordance with Quick.

The events occurring in the case of a blood vessel injury, which ultimately lead to blood coagulation and to the closing of the vessel wound, are simulated by adding blood or blood plasma from the patient to tissue thromboplastin in the presence of calcium, and measuring the time its takes for a coagulum to form. The tissue factor and lipids in the PT reagent activate factor VII, which in turn brings about, through factors X and V, the formation of thrombin from prothrombin (factor II). Thrombin cleaves the fibrinogen to insoluble fibrin which participates in the closing of the wound. The time it takes after the formation of thromboplastin plus calcium for the formation of a clot is a measure of the concentration or activity of the coagulation factors involved.

"Russell's Viper Venom" or the venom of Russell's viper (*Vipera russelli*) is abbreviated as RVV. In the field, Factor V-activating enzyme from Russell's viper venom, abbreviated as RVV-V, is used to allow activation of factor V directly without the need for other coagulation factors and in a calcium-independent manner, and is used to confirm factor V defects. This is for instance addressed in Tokunaga et al. "The Factor V-activating Enzyme (R VV-V) from Russell's Viper Venom" J. Biol. Chem. 263(33) pp. 17471-17481 (1988).

The test for establishing the suitability of a PT blood clotting assay in monitoring anticoagulant therapy according to the invention involves determining the assay's sensitivity for RVV-V addition. Without undue burden, the skilled person can readily assess the 'RVV-V sensitivity' of a blood clotting assay by determining the PT or clotting interval of a plasma sample containing a reversible direct factor Xa inhibitor with and without the presence of RVV-V. Although the skilled person may amend the test conditions to his own preference, as a non-limiting example a plasma concentration of 600 µl/ml of rivaroxaban may be provided as a model inhibitor. The RVV-factor V activator may for instance be obtained from Pentapharm Ag, Basel (Switzerland). For an effect to be noticed, it suffices to add 1 µl 1000U/ml RVV-V to 200 µl plasma. Notably, a straightforward test necessitating two measurements suffices. Otherwise, conventional PT assay conditions and sample preparations are unamended.

In one embodiment, the assay is considered "RVV-V sensitive" if the PT (in seconds) as measured for rivaroxaban in the above model upon addition of RVV-V is reduced with at least 30 %, more preferably at least 40 %, most preferably at least 50 %. A blood clotting assay is considered "RVV-V insensitive" if the PT in the above model is reduced with less than 30 %, more preferably less than 20 %, more preferably less than 10 %.

Alternatively, a PT assay is considered RVV-V sensitive if the addition of RVV-V reduces the PT for 600 µl/ml rivaroxaban with at least 20 seconds, preferably at least 30 seconds, more preferably at least 40 seconds, which is a significant change in such an assay. A PT assay exhibiting changes less than 10 seconds, preferably less than 5 seconds, is considered RVV-V insensitive.

In one embodiment, the assay is considered "RVV-V sensitive" if the PT ratio (compared to PT reference) at 600 µl/ml rivaroxaban drops from at least 3, preferably at least 3.5, more preferably at least 4.0 in absence of RVV-V to below 3, more preferably below 2.5, most preferably below 2.0 in presence of RVV-V.

It is added that the invention is not particularly limited to Factor V activator from the venom of Russell's viper, but the RVV-V provides a routine tool to assess the assay's ability to screen for reversible vitamin K antagonists. Here, RVV-V sensitivity may also be referred to as "Factor V activation sensitivity", to which it is linked.

Thromboplastins are prepared by providing a powdered thromboplastin source, converting the powdered thromboplastin source into a thromboplastin extract and preparing a useful PT reagent from the extract. Sources of thromboplastin include rabbit brain, human placenta, bovine brain, ox brain, human brain, and thromboplastin produced by recombinant DNA technology. Small changes in source and/or preparation method can have a large impact on the prothrombin properties of the reagent. Methods are for instance disclosed in US 4,416,812, US 5,254,350 and US 7,148,067.

In accordance with the present invention, the thromboplastin reagent may be provided in dried form, by freeze-drying, spray-drying, or other suitable protein drying methods. The PT reagents referred to in the text are understood to mean the composition containing the Tissue Factor itself, but also one or more of the aforementioned other components such as lipids, preservatives, stabilizers and/or calcium salts, e.g. CaCl₂. The thromboplastin reagent also contains phospholipids.

It is particularly preferred to use blood clotting assays resulting in a prolonged PT of at least 40 seconds in a test sample containing a plasma concentration ranging between 200 and 600 µl/ml of rivaroxaban. As shown in the examples, the blood clotting assay commercially sold as 'Triniclot PT Excel S' or 'Simplastin Excel S' (BioMerieux Inc. NorthCarolina) are particularly suited. Rivaroxaban-prolonged PT of 80 seconds was reduced in the presence of RVV-V to about 30 seconds for 200 - 600 µg/ml rivaroxaban. Additionally, Simplastin Excel S conveniently shows linear behaviour within the the therapeutic range, where other curves flatten at the higher end. The therapeutic range is between 1.0 and 5.0, which corresponds to the coumarin INR window which is a reference in the field.

The assay of the present invention may be used to monitor any reversible anticoagulant drug therapy involving reversible direct factor Xa inhibitor(s), i.e. to monitor any anticoagulant drug therapy comprising reversible direct factor Xa inhibitor(s), preferably at least rivaroxaban.

As explained above, in pre-operative screening it is necessary to distinguish between coumarins and reversible direct factor Xa inhibitors, in order to assess when the patient's blood is cleared from anticoagulants. Bleeding risks are thus minimized. Obviously, a singular test is not sufficient to measure two variables, i.e. the observation of a prolonged PT in a RVV-V sensitive assay cannot directly be linked to either anticoagulant. To that purpose, the inventors have developed a kit containing at least two containers, wherein a first container comprises an RVV-V sensitive PT reagent as explained above, and a second container comprises either Factor V-activating enzyme from Russell's viper venom (RVV-V), or RVV-V insensitive PT reagent(s), in either case optionally provided as a mixture with an RVV-V sensitive reagent. A non-limiting but preferred mixture is used in the accompanying examples, wherein said first container contains Simplastin Excel S, and said second container contains Innovin. For Simplastin Excel S clear RVV-V sensitivity of the PT observed for rivaroxaban has been demonstrated in example 1, while Innovin showed no RVV-V sensitivity and no effect on rivaroxaban alone.

The containers may be provided as separately packaged, or groups of the components may be packaged into 2 or more packages. Some or all of the components may be provided in dried forms, and other components provided in saline or a physiological buffer.

In one embodiment, the kit of the invention is a combination of parts of existing kits, with the restriction that the novel combination is not an arbitrary selection of existing blood clotting assays but involves a selection of two thromboplastins exhibiting RVV-V sensitive and insensitive PT in the afore-described model, respectively. Alternatively, said second container could contain a mixture of an RVV-V sensitive and an RVV-V insensitive PT reagent. In such embodiment, the weight ratio preferably varies between 3:1 and 1:3, preferably between 2:1 and 1:2. PT reagents may be used as provided commercially, without further amendment. Preferably the thromboplastin reagents contain calcium salts, or the calcium salts may be added just prior to use of the reagent. The calcium salt may be provided with the PT reagents in the kit, with each part separately packed, optional with the PT reagents in dry form. Calcium may be added as CaCl₂.

In another embodiment, the second container contains a stock solution of RVV-V which could be used in a PT measurement with the PT reagent provided in the first container, or, alternatively, the second container contains an RVV sensitive PT reagent (identical to or different from the PT reagent provided in the first container) and RVV-V, thus rendering the assay factor V insensitive. It is considered within the skilled person's daily routine to determine the concentrations of RVV-V most convenient to prepare such an assay. As a guidance, 1 µl 1000U/ml RVV-V to 200 µl plasma is found effective to cancel the sensitivity of a PT assay such as Simplastin Excel S.

The kit may be applied in a method for detecting and/or identifying reversible direct factor Xa inhibitor(s), wherein said method involves measuring a first PT and a second PT on the patient's blood plasma in a conventional manner, and comparing the first and second prothrombin times with one another. If a prolonged blood clotting time is observed in a PT assay using the RVV-V sensitive PT reagent provided in the first container, it can be concluded that either irreversible coagulation inhibitors or reversible direct factors Xa inhibitors may be present. An additional measurement is deemed necessary to identify the inhibitor, using the contents of the second container. If the patient has taken reversible direct factor Xa inhibitors, this would be reflected in a reduced PT when assaying with either an RVV-V insensitive thromboplastin reagent or a RVV-V sensitive thromboplastin reagent to which RVV-V has been added. In the latter case, the thromboplastin sensitivity for factor Va formation has been removed. If however the PT is not affected, the inhibitor(s) is/are of the irreversible kind.

### EXAMPLES

The experiments here below were performed with pooled human citrated plasma which was made from a minimum of individual contributions of 6 apparently healthy volunteers. Blood was collected ( 9 vol blood/ 1 volume 0.11 M citrate) in tubes and mixed directly after withdrawal. The Blood was cooled by submersion in ice water and centrifuged in a cooled, swing-out centrifuge for at least 20 minutes at 2000 x g. Subsequently, plasma was carefully separated from the cells and collected in an intermediate , cooled plastic tube. The collected plasma was mixed and centrifuged similarly after which platelet-poor-plasma was collected, aliquoted and after snap-freezing stored at -60 °C or below.

### Example 1a. Effect of rivaroxaban on blood clotting assays

Different PT assays were used using PT assays commercially available as Simplastin excel S (BioMerieux), Innovin (Dade Behring), Neoplastin CI plus (Stago), Recombiplastin (Instrumentation Laboratories) and Thromborel S (Dade Behring). In each case The reagent consisted of Tissue factor, lipid and CaCl₂. Tests were performed in a clotting analyser (AMAX Destiny, Kordia Lifesciences, Leiden, NL). In each case a cuvette was filled with 100 µl plasma and incubated for 1 minute at 37 °C. Pre-warmed PT reagent was added (200 µl) and recording of clotting was started. The AMAX recorded blood clotting by reduction in speed of a turning rod (mechanical detection). The clotting times were typically between 9-15 seconds depending upon the reagent used.

For spiking experiments with rivaroxaban (Xarelto, from Bayer), the plasma sample was spiked with rivaroxaban at different concentrations between 200 and 600 µg/ml, and the clotting times were again recorded using the different PT reagents. The results are plotted in Figure 1.

Based on the results plotted in Figure 1 it was concluded that only Simplastin Excel S was able to detect the reversible anticoagulant, whereas others failed or showed insignificant prolongation of the PT.

### Example 1b. Effect of rivaroxaban on blood clotting assays

Example 1a was repeated with PT clotting assays Thromboplastin S (Biopool, through Trinity),PT fibrinogen (Instrumentation Laboratories), PT fibrinogen recombinant (Instrumentation Laboratories), Thromboplastin C plus ( Siemens) and Simplastin Excel (Biomerieux).

The results of example 1a and 1b are shown in Table 1 below.

**Table 1. Prothrombin times of rivaroxaban (in seconds)**

| **PT assay** | **0** | **200 µg/ml rivaroxaban** | **600 µg/ml rivaroxaban** | **Ratio range*** |
|---|---|---|---|---|
| Simplastin excel S | 16.1 | 41.0 | 80.8 | 1.0-5.0 |
| Innovin | 9.5 | 12.0 | 17.1 | 1.0- 1.8 |
| Neoplastin CI plus | 13.4 | 20.0 | 35.0 | 1.0-2.6 |
| Recombiplastin | 10.3 | 16.8 | 26.2 | 1.0-2.5 |
| Thromborel S | 15.6 | 21.1 | 26.7 | 1.0-1.7 |
| Thromboplastin S | 21.2 | - | 51.6 | 1.0-2.4 |
| PT fibrinogen | 22.1 | 38.5 | 62.7 | 1.0-2.8 |
| PT fibrinogen recombinant | 23.6 | 32.9 | 45.4 | 1.0-1.9 |
| Thromboplastin C plus | 23.8 | 44.3 | 73.5 | 1.0-3.1 |
| Simplastin Excel | 20.4 | 40.0 | 63.1 | 1.0-3.1 |

| | | | | |
|---|---|---|---|---|
| *Lower and upper limits of ratio determined as the ratio of the values at 200 and 600 µg/ml rivaroxaban compared to the reference value ("0"), respectively. | | | | |

Although a reasonable therapeutic range is still observed for Thromboplastin C plus and Simplastin Excel, it is only Simplastin Excel S that covers the complete 1.0-5.0 therapeutic range. Also, Simplastin Excel S shows a linear relationship.

### Example 2. Effect of RVV-V on PT

The PT results reported in example 1a for Simplastin Excel S and Innovin were compared to another PT assay using these PT reagents, however now in presence of RVV-V. Thereto, RVV-factor V activator (Russel's viper venom V) (Pentapharm AG, Basel, Switzerland) (lot 40980901/121-03) was dissolved to a stock of 1000U/ml.

In the test, just before adding plasma to the AMAX, 1 µl RVV-V stock was added to 200 µl plasma or rivaroxaban-spiked plasma. Otherwise, test conditions were identical to those of example 1a.

The results are shown in Figure 2. The blood clotting assays based on Innovin still reported no significant change in PT for rivaroxaban, neither with nor without RVV-V. However, the prolonged PT observed using Simplastin Excel S was completely vanished when adding RVV-V prior to the clotting experiment.

It was thus concluded that the suitability of the Simplastin Excel S-based clotting assay for detecting reversible direct factor Xa inhibitors was associated with the Factor V activation. This was found to be the rate-limiting step in the Simplastin PT assay. When removing this rate-limiting action (by adding the accelerating RVV-V) the assay's sensitivity to rivaroxaban was also gone.

### Example 3. Identifying rivaroxaban

The results obtained for Simplastin Excel S and Innovin were plotted together with the PT results obtained with a 1:1 mixture of the two reagents. The experiment shows the usefulness of a kit of parts according to an embodiment of the invention.

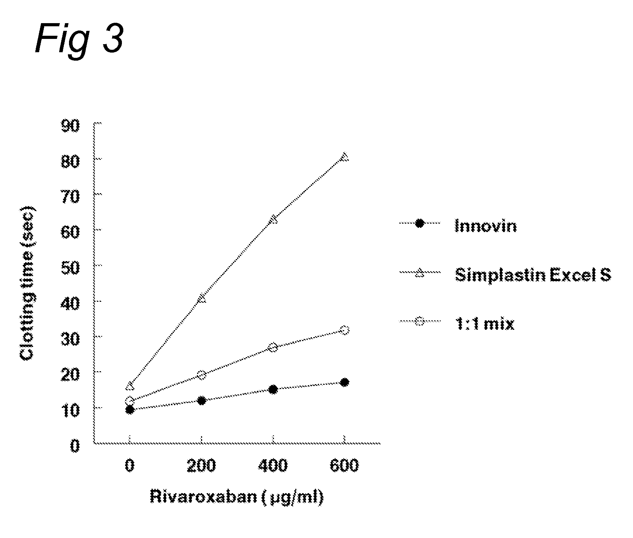

If a prolonged PT is observed with Simplastin Excel S assay, it is indicative of the presence of at least one of reversible and irreversible anticoagulants. By taking another measurement using a thromboplastin insensitive to the reversible anticoagulants, it can thus be established whether the patient's blood contained any reversible anticoagulants. In such case, the Innovin assay would show no prolonged PT at all. This case is in fact demonstrated in Figure 3.

## Claims

1. Use of an RVV-V sensitive blood clotting assay for monitoring anticoagulant therapy comprising reversible direct factor Xa inhibitor(s), and/or pre-operative screening for reversible direct factor Xa inhibitors.

2. Use according to claim 1, wherein said blood clotting assay is considered RVV-V sensitive if said assay on a plasma sample containing a reversible direct factor Xa inhibitor exhibits at least a 30 % decrease in prothrombin time if performed in the presence of Factor V-activating enzyme from Russell's Viper Venom (RVV-V), compared to the same assay without said Factor V-activating enzyme.

3. Use according to claim 1 or 2, wherein said blood clotting assay is considered RVV-V sensitive if said assay exhibits a change in PT ratio at 600 µl/ml rivaroxaban from at least 3.0 in the absence of RVV-V to below 3.0 in the presence of RVV-V.

4. Use according to any one of the preceding claims, wherein said reversible direct factor Xa inhibitor(s) comprises rivaroxaban.

5. Use according to any one of the preceding claims, wherein said blood clotting assay is Triniclot PT Excel S or Simplastin Excel S.

6. Use according to any one of the preceding claims, identifying or distinguishing said reversible direct factor Xa inhibitor(s) from irreversible vitamin K antagonists.

7. A blood clotting assay kit-of-parts, comprising
(a) a first container comprising RVV-V sensitive thromboplastin reagent;
(b) a second container comprising RVV-V insensitive thromboplastin reagent(s) or RVV-V, in either case optionally together with RVV-V sensitive thromboplastin reagent(s).

8. The kit-of-parts according to claim 7, wherein said second container contains a 3:1 - 1:3 mixture of said RVV-V sensitive thromboplastin reagent and said RVV-V insensitive thromboplastin reagent.

9. Use of the blood clotting assay kit-of-parts according to claim 7 or 8 for identifying reversible direct factor Xa inhibitors in a person's blood plasma.

10. Use according to claim 9, in pre-operative screening.

11. A method for identifying one or more reversible direct factor Xa inhibitors in a person's blood plasma, wherein said method involves a) measuring a first prothrombin time using a RVV-V sensitive thromboplastin reagent, b) measuring a second prothrombin time using an RVV-V insensitive thromboplastin reagent, a mixture of RVV-V sensitive and insensitive thromboplastin reagents, or an RVV-V sensitive thromboplastin reagent which has been made insensitive through addition of RVV-V, and c) comparing said first and second prothrombin times.
